# EUROPEAN PATENT APPLICATION

(11) **EP 2 698 111 A1**
(43) Date of publication of application: **19.02.2014**
(21) Application number: 12770745.3
(22) Date of filing: 29.02.2012
(51) Int. Cl.: A61B 5/1455, A61B 10/00

(54) **BIOPHOTONIC MEASUREMENT DEVICE, BIOPHOTONIC MEASUREMENT DEVICE OPERATING METHOD, AND BIOPHOTONIC MEASUREMENT DATA ANALYSIS AND DISPLAY METHOD**

(30) Priority: 15.04.2011 JP 2011091363
(71) Applicant: Hitachi Medical Corporation, Chiyoda-ku Tokyo 101-0021 (JP)
(72) Inventor: TANII, Michiyo, Tokyo 101-0021 (JP)
(74) Representative: MERH-IP Matias Erny Reichl Hoffmann
(86) International application number: PCT/JP2012/055108
(87) International publication number: WO 2012/140966

(57) **Abstract**

Result data indicating distribution of a biological metabolite concentration of a subject are generated by using biophotonic measurement data obtained with a biophotonic measurement device 10, whether the result data satisfy an arbitrary extraction condition concerning biophotonic measurement data set beforehand or not is judged, the result data are distinguishably displayed according to the results of the judgment, and thereby failure of extraction of measurement data of characteristic channels from result data obtained from as many as several tens of channels is prevented without depending on subjectivity of measurement operator.

## Description

### Technical Field

The present invention relates to a biophotonic measurement device, a method for operating a biophotonic measurement device, and a method for analyzing and displaying biophotonic measurement data, especially a technique for displaying results of a plurality of channels obtained by such measurement.

### Background Art

Patent document 1 discloses an example of biophotonic measurement device for measuring with a plurality of channels and imaging blood circulation, hemodynamics, and change of hemoglobin amount in the inside of a living body. Biophotonic measurement has a characteristic that it provides more superior time resolution, but poorer spatial resolution in measurement of a reaction of living body, especially a reaction concerning brain activity, compared with measurement with other devices for measuring such a reaction. However, techniques for improving the weak point of the biophotonic measurement, i.e., spatial resolution, have also been proposed in recent years. Patent document 2 discloses an example of technique for improving spatial resolution in measurement of a reaction concerning brain activity.

By the way, with progress of biophotonic measurement techniques, researches are conducted in various fields by utilizing such biophotonic measurement devices, which enable convenient and non-invasive measurement. For example, Non-patent document 1 discloses researches in the field of psychiatry and researches in the field of cerebral nerve. In order to fully utilize data obtained by biophotonic measurement in such researches, "analytical work" is important.

Therefore, Patent document 3 discloses an example of device additionally comprises analytical programs effective for these researches disclosed so far. Items to be noted in the analytical work include time until data start to change (time after a subject starts a task until change of the amount of hemoglobin appears), and time series change of such data. Patent document 4 describes examples of such notable items as mentioned above. It is considered that, as described above, not only improvement of measurement functions themselves of biophotonic measurement devices, but also functions for assisting in the analytical work will become more important in the future.

### Prior art references

### Patent document

Patent document 1: Japanese Patent No. 3796086
Patent document 2: Japanese Patent Unexamined Publication (KOKAI) No. 2006-325659
Patent document 3: Japanese Patent Unexamined Publication (KOKAI) No. 2009-000230
Patent document 4: WO07/135993

### Non-patent document

Non-patent document 1: Masato Fukuda, MEDIX, VOL. 39, 4-10, 2003

### Disclosure of the Invention

### Object to be Achieved by the Invention

By the measurement with a biophotonic measurement device, it is possible to monitor change of hemoglobin amount at measurement sites of several tens of channels at every 0.1 second or so as the minimum time frame. As operations following the measurement, any characteristic observed in the obtained result waveform or topography image is specified, and analysis is performed to obtain a conclusion. However, any routinized procedure has not been established for this analysis at present. Although several devices comprising programs for exclusive use for performing effective measurement and analysis are mentioned in the aforementioned researches, they cannot serve as general analytical means usable for all the kinds of measurements, and therefore usability thereof is limited. Moreover, although there are also programs enabling more general statistical analysis, they require a precondition that a certain characteristic must be specified beforehand in order to perform such analysis as mentioned above, for example, a statistical processing should be performed for "a group of data showing a certain common characteristic" or "data of channels showing a certain common characteristic". Furthermore, when analysis including statistical processing is performed for a certain time frame, the objective time frame of the analysis must also be decided beforehand. Therefore, as the preparative operation that must be first performed after the measurement is performed by a measurement operator and before a statistical processing or the like is performed, first of all, channels showing a certain characteristic are determined and extracted in time series. Then, the subsequent analytical operations (statistical processing etc.) are performed for data of the extracted channels.

The aforementioned preparative operation has a problem that the determination is performed by the measurement operator by visually seeing displayed results, that is, it greatly depends on the subjective viewpoint of the operator. That is, the measurement operator extracts characteristic data from the temporally changing result data obtained with a time resolution for 0.1 second from as many as several tens of channels by visual inspection, and therefore data that must essentially be extracted may be overlooked. It also has a problem that if the data are obtained by performing the measurement a plurality of times, and data showing a certain characteristic are extracted from the obtained data, the judgment criteria of the measurement operator may change in every measurement.

Furthermore, if the result is shown only as time series data as mentioned in Patent document 4, it is easy to generally and intuitively determine whether there is a reaction or not, but attentions may be paid to only a significant reaction, and data to be actually found as essentially notable by more precise analysis may be overlooked. Moreover, also depending on the display mode of the data, those that must be extracted may be overlooked. Furthermore, there is also a problem that it is difficult to intuitively determine change of data.

The present invention was accomplished in light of the aforementioned problems, and an object of the present invention is to provide a technique concerning display of biophotonic measurement data that can prevent failure of extraction of measurement data of characteristic channels from result data obtained from as many as several tens of channels without depending on subjectivity of measurement operator.

### Means for Achieving the Object

The present invention relates to a technique of setting an extraction condition for extraction of a characteristic portion of measurement result data (also referred to as "biophotonic measurement data") as the first step of the analysis stage in order to assist a measurement operator in such extraction, performing judgment in time series according to the condition, and displaying data satisfying the condition in an easily understandable manner.

More specifically, according to the present invention, by using biophotonic measurement data obtained with a biophotonic measurement device comprising an irradiation and measurement part for irradiating near-infrared light on a measurement site of a subject, measuring transmitted light intensities of the near-infrared light at a plurality of measurement points of the subject, and outputting signals corresponding to the transmitted light intensities at the measurement points as the biophotonic measurement data of the measurement points, and a display control part for displaying result data indicating distribution of biological metabolite concentration of the subject based on the biophotonic measurement data, the result data indicating distribution of biological metabolite concentration of the subject are generated, an arbitrary extraction condition for the biophotonic measurement data is set, whether each data satisfy the set extraction condition or not is judged, extraction of the biophotonic measurement data is performed on the basis of the result of the judgment, and the result data are distinguishably displayed on the basis of whether each of the data satisfies the condition or not.

That is, according to the present invention, a function for setting a condition for extracting data from the obtained measurement results (biophotonic measurement data) is provided. Items to be noted as the extraction condition include "time until data start to change", and "data change during task". Therefore, a condition concerning these items is set. Further, whether the data satisfy the condition or not is judged in time series, and those satisfying the condition are displayed among the results data with different colors according to data change.

The above function is a function for "assisting a measurement operator in the judgment by visual inspection", and sets an extraction condition and surely extracts a specific part of the data depending on the purpose.
Whether the extraction condition represents a biological activity or not does not relate to the function.

### Effect of the Invention

According to the present invention, there can be provided a technique concerning display of biophotonic measurement data, which can prevent failure of extraction of measurement data of characteristic channels from result data obtained from as many as several tens of channels without depending on subjectivity of measurement operator.

### Brief Description of the Drawings

Fig. 1 is a block diagram showing general configuration of a biophotonic measurement device of the present invention.
Fig. 2 is an explanatory drawing of a holder of the biophotonic measurement device, where (a) shows a state that the holder is attached to a subject and (b) a positional relationship of transmission optical fibers, reception optical fibers, and measurement channels on the holder.
Fig. 3 is a functional block diagram of a biophotonic measurement data analysis program 80.
Fig. 4 is a schematic diagram showing an example of topography image.
Fig. 5 is a schematic diagram showing an example of waveform graph
Fig. 6 is an explanatory drawing for explaining waveform map, where (a) shows an example of screen display of waveform map formed by mapping waveform graphs, and (b) shows a map of measurement channels corresponding to the waveform map of (a).
Fig. 7 is a schematic drawing showing an example of condition setting screen.
Fig. 8 is a schematic drawing showing an example of display of enlarged topography image
Fig. 9 is a schematic drawing showing an example of distinguishable display of enlarged topography image.
Fig. 10 is a schematic drawing showing an example of distinguishably displayed waveform map.
Fig. 11 is an explanatory drawing for explaining difference of results of judgment of states performed for waveform graphs by using different time frames.
Fig. 12 includes schematic drawings of distinguishable display in waveform graph unit.
Fig. 13 is a schematic drawing showing an example of distinguishable display in which one waveform graph is divided into a plurality of regions, and distinguishable display is performed in each divided region unit.
Fig. 14 is an explanatory drawing showing an example in which a plurality of waveform graphs are distinguishably displayed in rows.
Fig. 15 is a schematic drawing showing an example of actual display screen.

### Modes for Carrying out the Invention

The biophotonic measurement device of this embodiment comprises an irradiation and measurement part for irradiating near-infrared light on a measurement site of a subject, measuring transmitted light intensities of the near-infrared light at a plurality of measurement points of the subject, and outputting signals corresponding to the transmitted light intensities at the measurement points as biophotonic measurement data of the measurement points, and a display control part for displaying result data indicating distribution of a biological metabolite concentration of the subject based on the biophotonic measurement data, wherein the display control part comprises a result data generation part for generating the result data by using the biophotonic measurement data, an extraction condition setting part for setting an arbitrary extraction condition for the biophotonic measurement data, an extraction processing part for judging whether the biophotonic measurement data satisfy the extraction condition or not and performing extraction of the biophotonic measurement data on the basis of results of the judgment, and a distinguishable display processing part for distinguishably displaying the result data on the basis of whether the biophotonic measurement data satisfy the extraction condition or not.

The extraction condition setting part can set at least one of a ratio relative to a standard value of the biological metabolite concentration or value of the biological metabolite concentration, time passed from a standard time corresponding to a time point at which the subject starts a task, and time frame used for calculation of change of the biological metabolite concentration, as the extraction condition.

The extraction condition setting part can set a ratio relative to a standard value of the biological metabolite concentration or value of the biological metabolite concentration, and a time frame used for calculation of change of the biological metabolite concentration as the extraction condition, the extraction processing part can judge in which state the result data is, among states of a maintenance reaction where, during the time frame, ratio relative to the standard value or value of the biological metabolite concentration of the result data is not lower than the ratio relative to the standard value or value of biological metabolite concentration defined as the extraction condition, and the ratio or value continues, an increasing reaction where, during the time frame, ratio relative to the standard value or value of the biological metabolite concentration of the result data is not lower than the ratio relative to the standard value or value of biological metabolite concentration defined as the extraction condition, and the biological metabolite concentration increases, a decreasing reaction where, during the time frame, ratio relative to a standard value or value of the biological metabolite concentration of the result data is not lower than the ratio relative to the standard value or value of the biological metabolite concentration defined as the extraction condition, and the biological metabolite concentration decreases, and no reaction where, during the time frame, a state that ratio relative to the standard value or value of the biological metabolite concentration of the result data is lower than the ratio relative to the standard value or value of the biological metabolite concentration defined as the extraction condition continues, and the distinguishable display processing part can distinguishably display the result data by using display modes corresponding to the aforementioned states.

Further, the result data generation part can generate an enlarged topography image in which geometric figures surrounding the measurement points of the biological metabolite concentration of the subject are displayed by using one display mode for displaying whether the extraction condition is satisfied or not.

Further, the result data generation part can generate waveform graphs indicating temporal change of the biological metabolite concentration for the measurement points, and the distinguishable display processing part can distinguishably display waveform graphs satisfying the extraction condition and waveform graphs not satisfying the extraction condition.

Further, the result data generation part can generate a waveform map in which waveform graphs corresponding to the measurement points are arranged in accordance with arrangement of the measurement points, the extraction processing part can judge whether the extraction condition is satisfied or not for the waveform graphs, and the distinguishable display processing part can distinguishably display the waveform graphs in the waveform map on the basis of whether the extraction condition is satisfied or not.

1 Further, a time specification part for specifying an arbitrary time on the waveform graphs can be further provided, the extraction processing part can judge whether the extraction condition is satisfied or not at the specified time, and the distinguishable display processing part can distinguishably display the whole of the waveform graph.

Further, the extraction processing part can divide one of the waveform graphs into a plurality of regions, and judge whether the extraction condition is satisfied or not for each region, and the distinguishable display processing part can distinguishably display the regions of the one waveform graph on the basis of whether the extraction condition is satisfied or not.

Further, when the extraction processing part judges that the extraction condition is not satisfied, the distinguishable display processing part can display the result data by using a display mode corresponding to no satisfaction, and when the extraction processing part judges that the extraction condition is satisfied, the distinguishable display processing part can display the result data by using a display mode corresponding to the biological metabolite concentration.

Further, a measurement result storage part for storing biophotonic measurement data obtained by making different subjects perform the same task can be further provided, the result data generation part can generate the result data for the same time based on the time when the same task is started for each subject as the standard, the extraction processing part can judge whether the extraction condition is satisfied or not at that same time, and the distinguishable display processing part can display juxtapositionally the result data generated for the different subjects, and distinguishably display the result data on the basis of whether the extraction condition is satisfied or not.

Further, there can be further provided a first selection part for calculating integral value of the biological metabolite concentration of the subject during execution of the task, and selecting biophotonic measurement data giving such an integral value not lower than a predetermined value, and an emphasized display processing part for displaying result data based on the selected biophotonic measurement data with emphasis.

Further, there can be further provided a second selection part for selecting an arbitrary measurement point and selecting biophotonic measurement data of the measurement point, and an emphasized display processing part for displaying result data based on the selected biophotonic measurement data with emphasis.

Further, there can be further provided a result data storage part for storing at least one or more of display screens displaying the result data at an arbitrary time point, and a play back part for playing back a display screen stored in the result data storage part.

Further, the method for operating a biophotonic measurement device of this embodiment is a method for operating a biophotonic measurement device comprising the step of generating result data indicating distribution of biological metabolite concentration in a subject by using biophotonic measurement data obtained with a biophotonic measurement device comprising an irradiation and measurement part for irradiating near-infrared light on a measurement site of a subject, measuring transmitted light intensities of the near-infrared light at a plurality of measurement points of the subject, and outputting signals corresponding to the transmitted light intensities at the measurement points as the biophotonic measurement data of the measurement points, the method comprising the step of setting an arbitrary extraction condition for the biophotonic measurement data, the step of judging whether the set extraction condition is satisfied or not, and performing extraction of the biophotonic measurement data on the basis of results of the judgment, and the step of distinguishably displaying the result data on the basis of whether the extraction condition is satisfied or not.

Further, the method for analyzing and displaying biophotonic measurement data of this embodiment comprises the step of reading biophotonic measurement data of a subject, the step of generating result data indicating distribution of biological metabolite concentration in the subject by using the biophotonic measurement data, the step of setting an arbitrary extraction condition for the biophotonic measurement data, the step of judging whether the set extraction condition is satisfied or not, and performing extraction of the biophotonic measurement data on the basis of result of the judgment, and the step of distinguishably displaying the result data on the basis of whether the extraction condition is satisfied or not.

Hereafter, the biophotonic measurement device, method for operating a biophotonic measurement device, and method for analyzing and displaying biophotonic measurement data of this embodiment will be explained in more detail with reference to the drawings. The same numerical notations are used for components having the same functions, and repetitive explanations thereof are omitted.

### <General configuration>

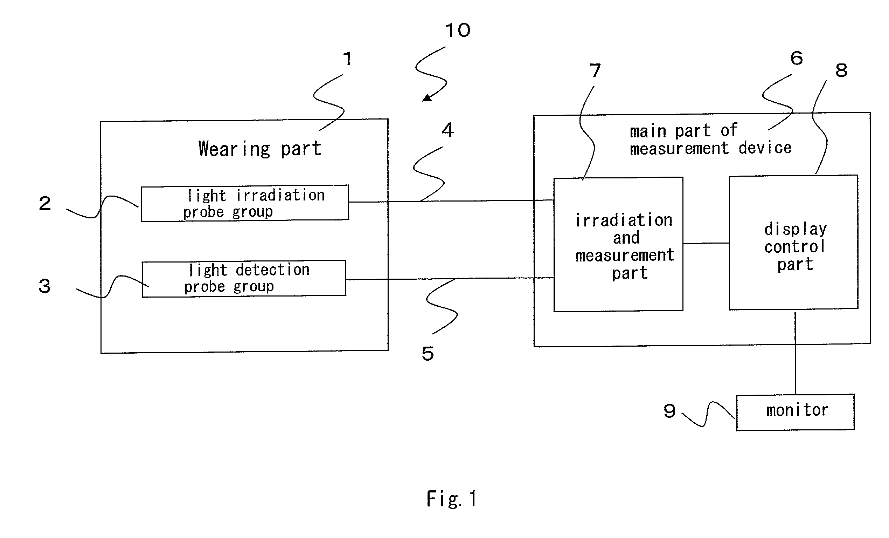

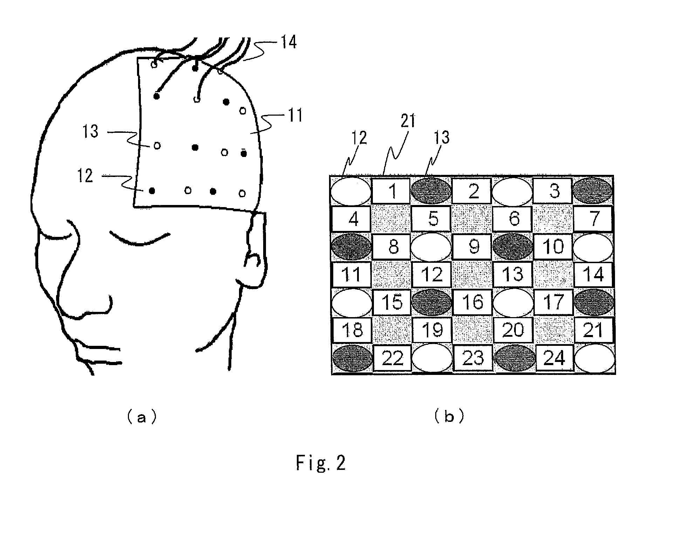

First, the general configuration of the biophotonic measurement device of the present invention will be explained with reference to Figs. 1 and 2. Fig. 1 is a block diagram showing the general configuration of the biophotonic measurement device of the present invention. Fig. 2 includes explanatory drawings of a holder provided in the biophotonic measurement device. Fig. 2 (a) shows a state that the holder is attached to a subject, and Fig. 2 (b) shows a positional relationship of transmission optical fibers, reception optical fibers, and measurement channels on the holder.

The biophotonic measurement device 10 is a device for measuring change of hemoglobin concentration in the brain by irradiating near-infrared light on a living body and detecting the light passed through the living body. As shown in Fig. 1, the biophotonic measurement device 10 comprises a wearing part 1 comprising a light irradiation probe group 2 and a light detection probe group 3, a main part 6 of the measurement device comprising the irradiation and measurement part 7 for controlling irradiation and detection of near-infrared light, and the display control part 8 for forming an image from biophotonic measurement data based on transmitted infrared light detected by the light detection probes and displaying the image. The wearing part 1 and the irradiation and measurement part 7 are electrically connected through an optical fiber group 4 for irradiation including a plurality of optical fibers for irradiation, and an optical fiber group 5 for detection including a plurality of optical fibers for detection.

The light irradiation probe group 2 includes a plurality of light irradiation probes for irradiating near-infrared light on a subject. The light detection probe group 3 includes a plurality of light detection probes for detecting the near-infrared light that passed through the subject. The light irradiation probes and the light detection probes are arranged in the form of a matrix on the holder 11, as shown in Fig. 2(a). Further, the light irradiation probes and the light detection probes are alternately arranged. In addition, the holder 11 may have a size and shape suitable for a part to be measured selected from various kinds of sizes and shapes.

When biophotonic measurement of a subject is performed, the holder 11 is attached on a portion of the subject to be measured (for example, head). In the holder 11, the optical fibers 12 which transmit the light to be irradiated, and the optical fibers 13 which transmit the transmitted light are arranged in a matrix of four rows each for length and width with intervals between adjacent optical fibers. Halfway points of adjacent optical fibers are defined as measurement points (also referred to as measurement channels). In the case of the holder 11, there are 24 of measurement channels 21. In this specification, hemoglobin concentration means any one or any combination of oxygenated hemoglobin concentration, deoxygenated hemoglobin concentration, and total hemoglobin concentration.

The subject wearing the holder 11 attached with the optical fibers in a certain state is given a certain task, and change of a biological metabolite concentration ("hemoglobin concentration" is used in this embodiment) observed during execution of the task from the biological metabolite concentration observed in the steady state is measured.

The irradiation and measurement part 7 generates near-infrared light (light having a wavelength of from visible to infrared region), transmits it to the light irradiation probe group 2, and measures amount of light detected by the light detection probe group 3. Further, the irradiation and measurement part 7 measures change of the hemoglobin concentration in the blood at each measurement point from the amounts of light detected by the light detection probe group 3.

The display control part 8 creates a topography image as a two-dimensional distribution image of the hemoglobin concentration, or waveform graphs showing temporary change of the hemoglobin concentration at the measurement points and a waveform map formed by two-dimensional mapping of the waveform graphs, on the basis of the results of the measurement performed by the irradiation and measurement part 7, and displays them on a monitor 9. The main part 6 of the measurement device comprises a computer including a processing part (CPU etc.), a storage part (ROM, RAM, hard disk, etc.), and a signal input and output part. Programs for realizing the functions of the irradiation and measurement part 7 and the display control part 8 are stored in the storage part of the computer.

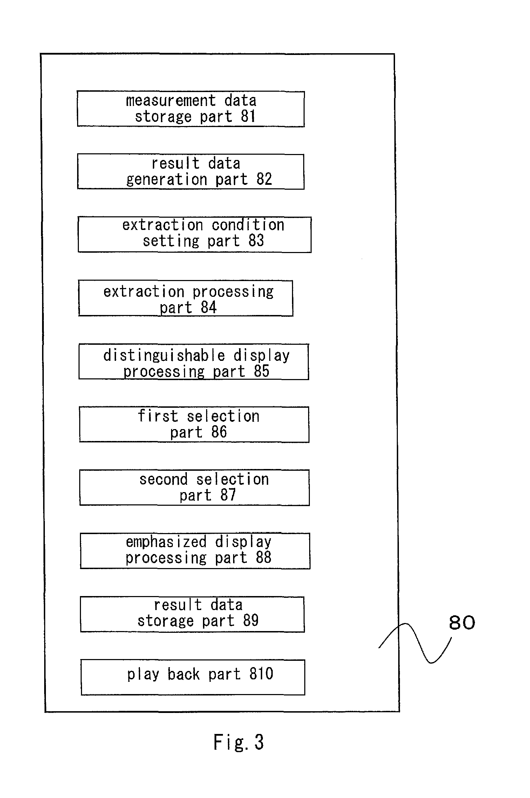

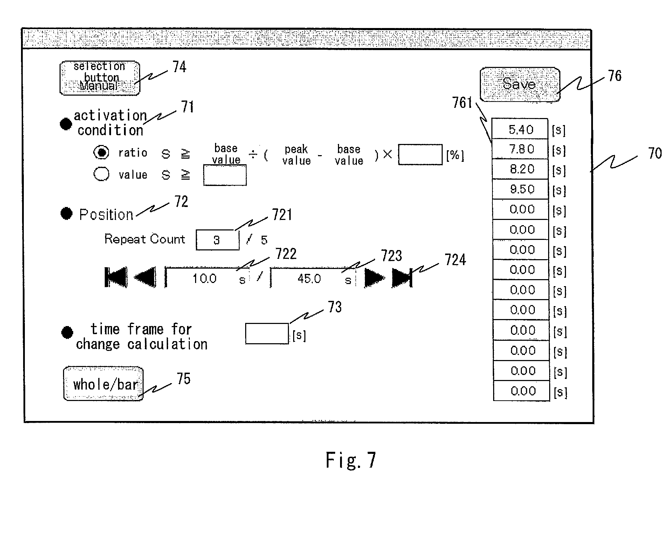

Fig. 3 is a functional block diagram of a biophotonic measurement data analysis program 80 stored in the display control part 8 shown in Fig. 1. The biophotonic measurement data analysis program 80 comprises a measurement result storage part 81 for storing data concerning measurement results of biophotonic measurement (henceforth referred to as "biophotonic measurement data") based on signals corresponding to transmitted light intensities sent from the light detection probe group 3 and received by the irradiation and measurement part 7, a result data generation part 82 for reading biophotonic measurement data from the measurement result storage part 81, and generating a topography image formed by two-dimensional or three-dimensional mapping of biological metabolite concentrations at measurement points (for example, deoxygenated hemoglobin concentration, oxygenated hemoglobin concentration, or total hemoglobin concentration), or waveform graphs showing temporal change of biological metabolite concentration at the measurement points by using a wave shape display mode for each measurement channel and a waveform map formed by mapping of the waveform graphs (these "topography image", "waveform graph" and "waveform map" are generically referred to as "result data") on the basis of the biophotonic measurement data, an extraction condition setting part 83 for starting and displaying a condition setting screen (refer to Fig. 7), which will be explained later, and receiving setting of an arbitrary extraction condition for biophotonic measurement data, an extraction processing part 84 for performing extraction of the biophotonic measurement data according to the set extraction condition, and judging whether the biophotonic measurement data satisfy the extraction condition or not, a distinguishable display processing part 85 for realizing distinguishable display on the basis of whether the extraction condition is satisfied or not, a first selection part 86 for calculating integral value of the biological metabolite concentration of the subject during execution of the task, and selecting biophotonic measurement data giving such an integral value not lower than a predetermined value, a second selection part 87 for receiving input of selection of arbitrary measurement points from a measurement operator, and selecting biophotonic measurement data of the measurement points, an emphasized display processing part 88 for displaying the measurement points selected by the first selection part 86 or the second selection part 87 with emphasis, a result data storage part 89 for storing at least one or more of display screens of result data at arbitrary time points, and a play back part 810 for playing back a display screen stored in the result data storage part 89.

The measurement result storage part 81 individually stores biophotonic measurement data of each subject. The data of each subject for each examination task are separately stored. Furthermore, the measurement result storage part 81 stores body marks schematically showing arrangement of the measurement points (measurement channels) with correlating them with the biophotonic measurement data.

In this embodiment, the biophotonic measurement device 10 carries the aforementioned biophotonic measurement data analysis program 80. However, the biophotonic measurement data analysis program 80 can also be carried on a personal computer or a workstation to constitute the biophotonic measurement device and display results of analysis of biophotonic measurement data. Further, in this embodiment, the aforementioned result data are used as the result data, but the data structure is not particularly limited, i.e., the data may be in the form of a list, table, graph, or the like of numerical data, so long as data indicating distribution state of the biological metabolite concentration are used. In addition, these result data can also be displayed by distinguishable display explained below.

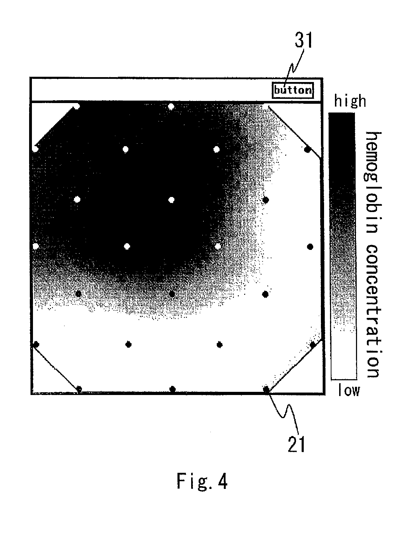

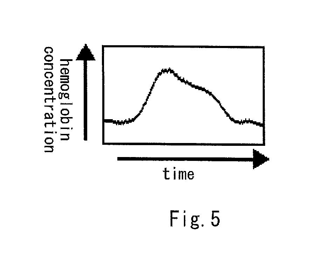

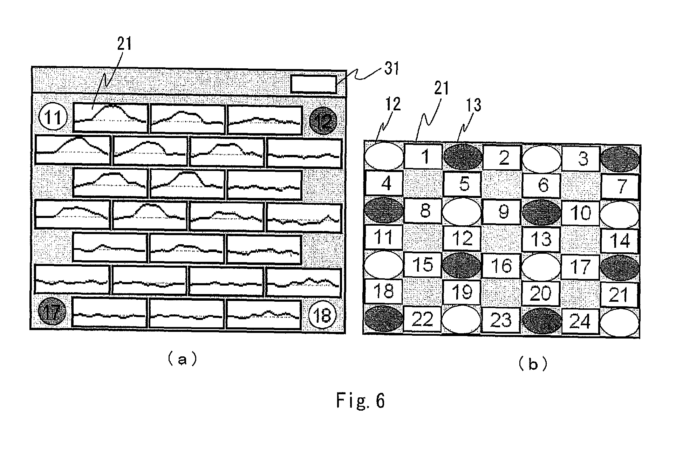

Hereafter, the result data using biophotonic measurement data will be explained with reference to Figs. 4 or 6. Fig. 4 is a schematic drawing showing an example of topography image. Fig. 5 is a schematic drawing showing an example of waveform graph. Fig. 6 includes explanatory drawings for explaining waveform map, Fig. 6(a) shows an example of screen display of a waveform map in which waveform graphs are mapped, and Fig. 6(b) shows a map of measurement channels corresponding to the waveform map of Fig. 6(a).

As the result data using biophotonic measurement data, there are mainly two kinds of data such as the topography image shown in Fig. 4 and those in the form of waveform shown in Fig. 5. These topography image and waveform are generated and displayed by the result data generation part 82.

As shown in Fig. 4, the topography image depicts the measurement channels 21 (equivalent to the measurement channels 21 shown in Fig. 2(b)) in accordance with the actual arrangement state, and represents the hemoglobin concentrations of the measurement channels 21 at a certain time point with shade of color. Portions between the measurement channels are depicted by using an interpolation algorithm.

The waveform graphs show change of the hemoglobin concentration at the measurement channels in time series for each measurement channel, in which the vertical axis indicates lapsed time, and the horizontal axis indicates the hemoglobin concentration, as shown in Fig. 5. Since a plurality of measurement channels are usually used, such waveform graphs as shown in Fig. 5 are displayed in the form of waveform map as shown in Fig. 6(a), in which the waveform graphs are arranged in accordance with actual arrangement of the measurement channels (refer to Fig. 6(b)). The waveform map of Fig. 6A is for a case where the number of measurement channels is 24, and displays 24 waveform graphs. The channel positions and the measurement points of the waveform map of Fig. 6(a) and the map of the measurement channel of Fig. 6(b) agree with each other, and in Fig. 6(b), between light irradiation probe positions 11 and 18 and light detection probe positions 12 and 17, measurement channels 1, 2, and 3, and measurement channels 22, 23, and 24 are arranged, respectively. Correspondingly, in the waveform map of Fig. 6(a), between the light irradiation probe position 11 and the light detection probe position 12, the waveforms showing temporal change of the hemoglobin amount at the measurement channels 1, 2, and 3 are arranged, and the waveforms showing temporal change of the hemoglobin amount at the measurement channels 22, 23, and 24 are arranged between the light irradiation probe position 18 and the light detection probe position 17. Processings for arrangement and display of the waveforms are also performed by the result data generation part 82.

If the topography image and the waveform graph map are compared, with the topography image display of Fig. 4, distribution of the hemoglobin amount can be intuitively understood, and total brain activities can be easily understood, but since it does not include temporal information, it is difficult to understand the temporal change. Further, since the shade of color indicating change of the hemoglobin concentration is precise, it is difficult to judge "whether there is a reaction" or not at a certain time point.

On the other hand, if such a waveform map as shown in Fig. 6 is used, it became easier to see a reaction site among the actual measurement positions, compared with a case of arranging such waveform graphs as shown in Fig. 5 from the top to the bottom in an order of measurement channels provided for convenience, and it can be said that this makes it possible to understand the whole brain activities in time series. However, it is not easy for a measurement operator to make judgment for waveforms measured with as many as several tens of channels by visual inspection.

The present invention is characterized by providing an additional function useful for visual measurement of the topography image and waveform graph map by a measurement operator. More specifically, an extraction condition for measurement results is set beforehand, and whether the condition is satisfied or not is distinguishably displayed. The following explanation will be made for a case where such a processing as mentioned above is used in a phase of analysis of biophotonic measurement data performed after examination for obtaining and storing the biophotonic measurement data performed by making a subject wearing the holder 11 execute a task. However, it may also be used for real time display of result data based on biophotonic measurement data obtained during execution of a task by a subject.

Hereafter, processing for setting extraction condition will be explained with reference to Fig. 7. Fig. 7 is a schematic drawing showing an example of condition setting screen 70.

The condition setting screen 70 principally comprises an activation condition setting part 71 for setting a condition for extracting biophotonic measurement data, especially a condition for classifying states of change of hemoglobin amount into those corresponding to the presence and absence of a reaction, and in the case of the presence of a reaction, those corresponding to the increasing reaction, maintenance reaction, and decreasing reaction, a time specification part 72 for setting an extraction condition utilizing lapsed time from the start of task (corresponding to the part indicated as "Position"), a "time frame for change calculation" setting part 73 for determining time frame used for judging presence of a reaction time, a "Selection button" 74 for selecting whether selection of measurement channel to be noted is performed automatically or manually, a "Whole/bar" switch button 75 for selecting whether the distinguishable display is used for the whole waveform graph or divided regions formed by dividing each waveform graph, a "Save" button 76 for inputting a direction for storing at an arbitrary time point (displayed screen), and a list column 761 for displaying a list of record times relative to the start time of the biophotonic measurement data, at which the aforementioned "Save" button 76 is operated.

With the activation condition setting part 71, a standard condition for judging presence or absence of a reaction determined by a measurement operator is set. For example, the standard value and the maximum value in the measurement are defined to be 0 and 1, respectively, and the condition is set by utilizing ratio so that if the value exceeds 40% of the maximum value, it is judged that there is a reaction (condition setting utilizing "ratio"), or the condition is set so that if the change of hemoglobin concentration exceeds 0.1, it is judged that there is a reaction (condition setting utilizing "value"). The extraction condition mentioned above is for assisting a measurement operator in the judgment, which has conventionally been performed on the basis of only visual inspection, and extraction condition should not include unduly complicated conditions. The reaction present state may be further classified into 3 kinds of states, i.e., (1) a state that measured value significantly increases from the previously measured value ("increasing reaction" mentioned above), (2) a state that the previously measured value is maintained ("maintenance reaction" mentioned above), and (3) a state that measured value significantly decreases from the previously measured value ("decreasing reaction" mentioned above). In addition to these three kinds of states, there is the state where the extraction condition is not satisfied ("no reaction" mentioned above), and therefore there are 4 kinds of states in total. In the following embodiments, distinguishable display is performed with display modes corresponding to these states, respectively.

The time specification part 72 (equivalent to the part of "Position") indicates lapsed time 722 from the start of the task, of which time is indicated as 0, and time to the following task, or time 723 at the end of the measurement. By operating time control bars 724, the time from the start of the task to generation of the result data can be changed. In the box 721 of "Repeat Count" part, the number of repetition time of the task is inputted. The lapsed time before the first repetition is indicated with the minus sign. This is for most directly representing "time until data change" as an item to be noted. For example, the numerical values displayed as shown in Fig. 7 mean "the third task among five times of the task, and hemoglobin data after 10 seconds passed within 45 seconds from the third task to the start of the fourth task", and a hemoglobin concentration state at this time point is depicted by the result data (such a topography image as shown in Fig. 4, or such waveform graphs as shown in Fig. 5, and such a waveform map as shown in Fig. 6).

With the time frame for change calculation setting part 73, the time frame from the previous time to the present time is set as an item to be set for calculating the four kinds of states of reaction mentioned above. A time point going back from the lapsed time 772 by the time frame determined by the time frame set in the time frame for change calculation setting part 73 is defined as the "previous time", and the change is calculated by using the hemoglobin value at the time point of the previous time as the previously measured value.

The details of the "Selection button" 74, the "Whole/bar" switch button 75, the "Save" button 76, and the list column 761 will be explained later.

The condition setting screen 70 shown in Fig. 7 is commonly used for the first to fourth embodiments. However, parts for functions unnecessary for a specific embodiment are displayed in an inoperable state in the screen for the specific embodiment. For example, the "Whole/bar" switch button 75, the "Save" button 76, and the list column 761 are unnecessary for displaying an enlarged topography image, which will be explained later, and therefore, in such a case, these buttons are displayed in a state that they cannot be operated.

The extraction condition setting part 83 receives the extraction condition set in the condition setting screen 70 shown in Fig. 7. The extraction processing part 84 performs extraction of biophotonic measurement data according to the extraction condition, for example, performs extraction processing of biophotonic measurement data in the set time frame, and judges whether the extracted biophotonic measurement data satisfy the set condition or not. The judgment concerning whether the extracted biophotonic measurement data satisfy the set condition or not mentioned above includes the judgment concerning the reaction states described above. Then, the distinguishable display processing part 85 changes display color of the topography image and waveforms according to whether the condition is satisfied or not or the judged state.

Hereafter, examples of the screen display corresponding to an extraction condition set in the condition setting screen will be explained.

### <First embodiment>

The first embodiment is an embodiment in which a topography image or waveform graph of a noted channel is displayed with emphasis for biophotonic measurement data of one subject.

### (Additional function for topography image)

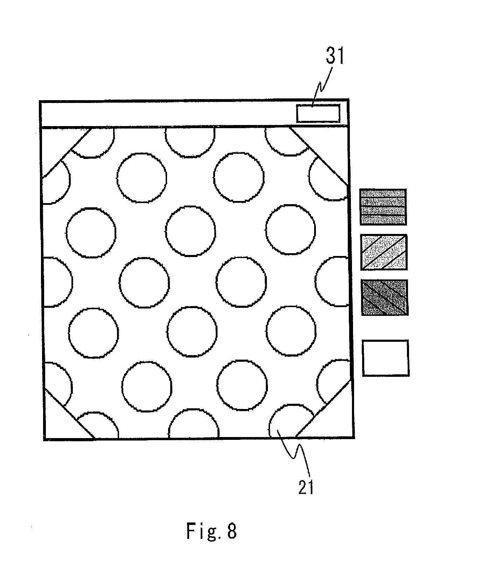

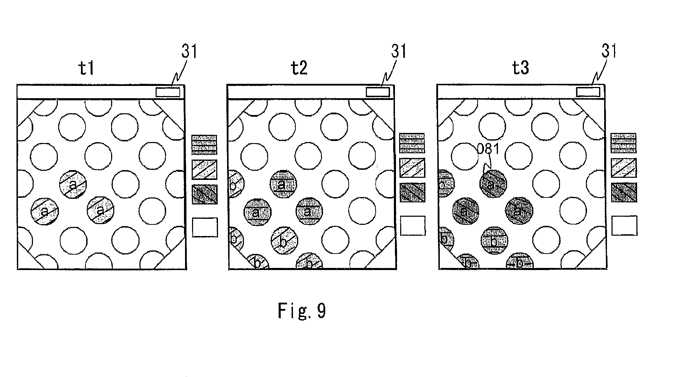

Additional functions for such a topography image as shown in Fig. 4 will be explained below with reference to Figs. 8 and 9. Fig. 8 is a schematic drawing showing an example of display of an enlarged topography image. Fig. 9 includes schematic drawings showing examples of distinguishable display of an enlarged topography image.

On the display screen of the topography image of Fig. 4, a setting button 31 is provided. If the setting button 31 is operated, the condition setting screen shown in Fig. 7 starts as another window, and the topography image of Fig. 4 is switched to such an enlarged topography image as shown in Fig. 8, in which the channels are displayed with enlargement. In the enlarged topography image of Fig. 8, the measurement channels are displayed with enlargement, and geometric figures representing the measurement channels 21 (circles in Fig. 8) are each colored with a color corresponding to change of state of each measurement channel.

By confirming the enlarged topography image of Fig. 8 together with the lapsed time 722, change of the concentration relative to time change can be more easily understood. In Fig. 8, the maintenance reaction is indicated with lateral stripes, the increasing reaction is indicated with oblique lines from lower left to upper right, the decreasing reaction is indicated with oblique lines from lower right to upper left, and no reaction is indicated with no oblique line and no stripe (the same shall apply to Figs. 9, 12, and 13), for convenience of the explanation. When the state changes, the color of the geometric figures corresponding to the measurement channels 21 of Fig. 8 changes in the display, and therefore the measurement operator easily notice that change, and can immediately read the lapsed time at that time point. This display scheme is designed by focusing on the presence and absence of reaction, and is specialized for display of them, and therefore it makes the judgment easier. For example, it becomes possible to judge that a channel in which reaction is seen within 0.3 second of the lapsed time 722 shows a quick reaction, and a channel in which reaction is seen after the lapsed time of 1 second or longer shows a slow reaction.

Fig. 9 shows an example of the distinguishable display using an enlarged topography image, and shows enlarged topography images at three time points of t1, t2, and t3. At the time point t1, the measurement channels a are in the state of the increasing reaction. At the time point t2, the measurement channels a are in the state of the maintenance reaction, and the measurement channels b are newly in the state of the increasing reaction. At the time point t3, the measurement channels a are in the state of the decreasing reaction (081), and the measurement channels b are in the state of the maintenance reaction. Instead of the aforementioned example of the display, only the measurement points in a desired state may be displayed with geometric figures, and the other measurement points may be displayed by using usual display scheme corresponding to biophotonic metabolite concentration. For example, in the topography image, only the measurement points at which the condition is not satisfied may be distinguishably displayed with a display scheme using geometric figures corresponding to non-satisfaction, and the measurement points at which the condition is satisfied may be displayed by using a display scheme corresponding to biophotonic metabolite concentration.

### (Additional function for waveform map)

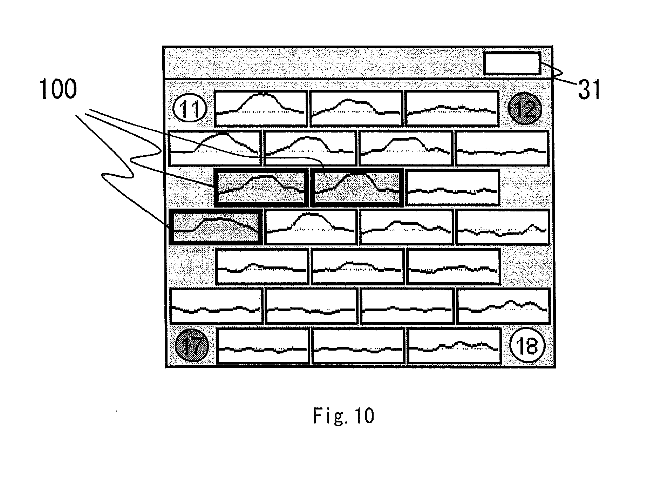

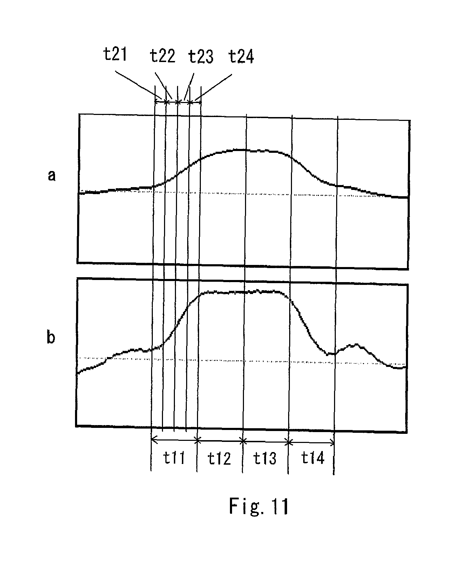

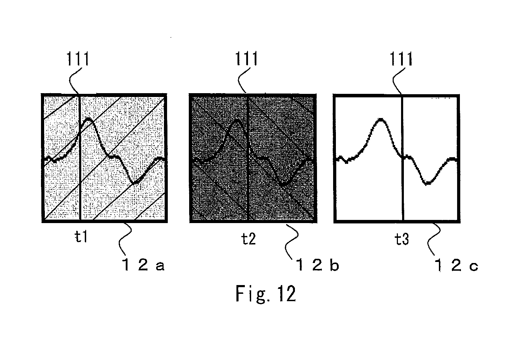

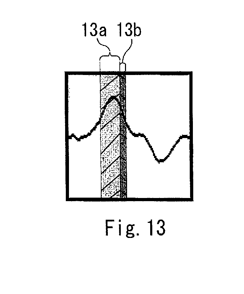

Additional functions for such a waveform map as shown in Fig. 6 will be explained below with reference to Figs. 10 to 13. Fig. 10 is a schematic drawing showing an example of distinguishably displayed waveform map. Fig. 11 is an explanatory drawing for explaining difference of results of judgment of states performed for waveform graphs by using different time frames. Fig. 12 is a schematic drawing showing an example of distinguishable display in waveform graph unit. Fig. 13 is a schematic drawing showing an example of distinguishable display in which one waveform graph is divided into a plurality of regions, and distinguishable display is performed for each divided region unit.

As in the case of Fig. 4, a setting button 31 is provided on the display screen of the waveform map of Fig. 6(a). If the setting button 31 is operated, the condition setting screen shown in Fig. 7 starts, and the channels noted throughout the measurement are displayed with emphasis, and thereby made visually conspicuous. There are two kinds of ways for selecting the channels to be made conspicuous. One is a method of automatically selecting channels showing large waveform increase amount during execution of the task (it is equivalent to the integral value of the hemoglobin amount, and displayed as an area of waveform graph in the case of the waveform graph of Fig. 6(a)). The other is a method of manually selecting arbitrary channels.

As for use of the former, it is used for reading sites reactive to the task on the basis of measurement results, and finding any characteristic from them. The latter is used for performing the task with preliminarily assuming channels considered to be reactive to the task. For example, when a word recollection task (verbal fluency test) is performed, it is presumed that the frontal lobe reacts, and measurement channels locating at the forehead are manually selected. Further, when a finger tapping task is performed, it is presumed that the temporal lobe reacts, and measurement channels locating at the temporal region are manually selected.

It is effective for investigation of waveform graphs to, in addition to be always aware of such noted channels as mentioned above, employ approaches of observing at what time reactions occur at the noted channels using further finer time unit, whether any characteristic waveforms are generated at channels other than the noted channels, or the like. However, it is considered that, for paying attention to the noted channels among as many as several tens of channels of measurement sites, it is necessary to display them with emphasis.

Therefore, as an example of the means for displaying the noted channels with emphasis, the "Selection button" 74 is provided on the condition setting screen 70 of Fig. 7. Whenever the "Selection button" 74 is operated, the method for selecting the noted channels is changed between automatic and manual selections, and which selection method is selected is displayed on the "Selection button" 74.

In the case of the automatic selection, the first selection part 86 calculates waveform increase amount of each channel, and selects a channel showing the largest increase amount. Then, the emphasized display processing part 88 realizes emphasized display by using a different color, blinking or the like for the display of waveform graphs of the selected channel.

In the case of the manual selection, the measurement operator selects a channel for which emphasized display is desired by using a mouse, and the second selection part 87 selects the waveform graph of the channel. Then, the emphasized display processing part 88 realizes emphasized display by using a different color, blinking or the like for the display of waveform graph of the selected channel.

An example of the screen displaying waveform graphs displayed with emphasis is shown in Fig. 10. In Fig. 10, three of graphs 100 are color-displayed with emphasis. By visually conspicuously displaying the waveform graphs, the operator can be made easier to be always aware of the channels thereof, and possible to concentrate on other judgment works.

Functions and setting methods of other conditions that can be set with setting means other than the "Selection button" 74 provided in the condition setting screen 70 of Fig. 7, for example, the activation condition setting part 71, the lapsed time display part 72, and the time frame for change calculation setting part 73, are the same as those explained for the additional function for topography image described above. Further, the following operations are performed also as explained for the additional function for topography image described above, that is, extraction processing for extracting channels satisfying the condition and judgment processing for whether the set condition is satisfied or not are performed by the extraction processing part 84, and in accordance with the results of the judgment, the distinguishable display processing part 85 displays the waveform graphs of the channels with successively changing background color of the waveform graphs.

The technical meaning of magnitude of the value set with the time frame for change calculation setting part 73 will be explained below. If the time frame is set to be shorter, data of a transient state are focused, and if it is set to be longer to a certain extent, whether the state is generally an increasing state or decreasing state for one task, whether a reaction for a task is continuous or not, and so forth are focused.

For example, if the time frame is set to be t11 shown in Fig. 11 with the time frame for change calculation setting part 73 shown in Fig. 7, it can be seen that, as the general tendency of both the waveforms a and b, the reaction state is a state of positive change during t11 before t12 (duration t11), that state is maintained (durations t12 and t13), and then it is a state of negative change (duration t14). However, if the time frame is set to be t21 shown in Fig. 11 with the time frame for change calculation setting part 73 shown in Fig. 7, there are obtained results that, during t21 to t24 (namely, during a transient state), the waveform a shows no change, but the waveform b shows positive change, although the results may vary depending on setting of a threshold value, and it can be seen that the waveform a shows a more gradual change compared with the waveform b.

If the condition is set on the condition setting screen 70 shown in Fig. 7, the results of the setting are reflected in the graphs in the form of waveform map shown in Fig. 6 so that the graphs are displayed in different colors. That is, the four kinds of states described above, i.e., (1) significant increase compared with the previously measured value, (2) maintenance of the previously measured value, (3) significant decrease compared with the previously measured value, and (4) no satisfaction of condition/no reaction, are distinguishably displayed. There are two types of the display, and they can be switched with the "Whole/bar" button 75.

Fig. 12 shows an example of the display in a state that the whole display is selected by operating the "Whole/bar" button 75. On waveform graphs 12a, 12b, and 12c (these are all the same waveform graphs, that is, they are waveform graphs obtained with the same measurement channel for the same subject), a time bar 111 indicating display time (lapsed time 722 shown in Fig. 8) is superimposingly displayed, and the extraction processing part 84 judges the state at that time. And the distinguishable display processing part 85 depicts the whole background of the waveform graph using a background color corresponding to the state at the time obtained as a result of the judgment. The time bar 111 can be moved by operating the time control bars 724 or directly pointing an arbitrary waveform graph with a mouse.

By changing the time by continuously operating the time control bars 724 in the display scheme shown in Fig. 12, data can be mechanically extracted in time series according to the judgment criteria arbitrarily set by the measurement operator. Further, the change thereof is also depicted with different colors, and therefore distinction becomes easy. If a site to be noted is set beforehand for a certain task, time required for most part of the noted part to come to be in a state for extraction after the task is given, and so forth can be easily understood.

In Fig. 12, an increasing reaction is observed at the time t1, and therefore the background color of the whole waveform graph 12a is a background color corresponding to the increase reaction. At the time t2, a decreasing reaction is observed, and therefore the background color of the whole waveform graph 12b is a background color corresponding to the decreasing reaction. At the time t3, there is no reaction, and therefore the background color of the whole waveform graph 12c is a background color corresponding to no reaction. Although not shown in Fig. 12, when the maintenance state is observed at the time specified on the waveform graph, the whole waveform graph is depicted with a background color corresponding to the maintenance reaction.

Fig. 13 shows an example of the display in a state that the bar display is selected by operating the "Whole/bar" button 75. In the case of the display shown in Fig. 13, the extraction processing part 84 divides the waveform graph into sections thereof for a value of time frame set in the time frame for change calculation setting part 73, and a state at a time within each time frame is judged for each section. The distinguishable display processing part 85 divides the waveform graph into regions of the aforementioned time frame, and distinguishably displays the divided regions 13a and 13b using a background color corresponding to the state at that time.

In the case of the display scheme of Fig. 13, data change of each channel can be more easily visually understood, compared with the case of displaying only waveform without the distinguishable display, and correct extraction in accordance with the judgment criteria is enabled.

According to this embodiment, it becomes possible to mechanically extract a specific part of specific channels satisfying judgment criteria set for temporally changing biophotonic measurement data. Thus, since it is not visual extraction by human, the influence of inconsistency of judgment and omission of extraction are prevented, and it becomes easy to generally see brain activities in time series. Therefore, biophotonic measurement data can be surely utilized in the following analysis.

### <Second embodiment>

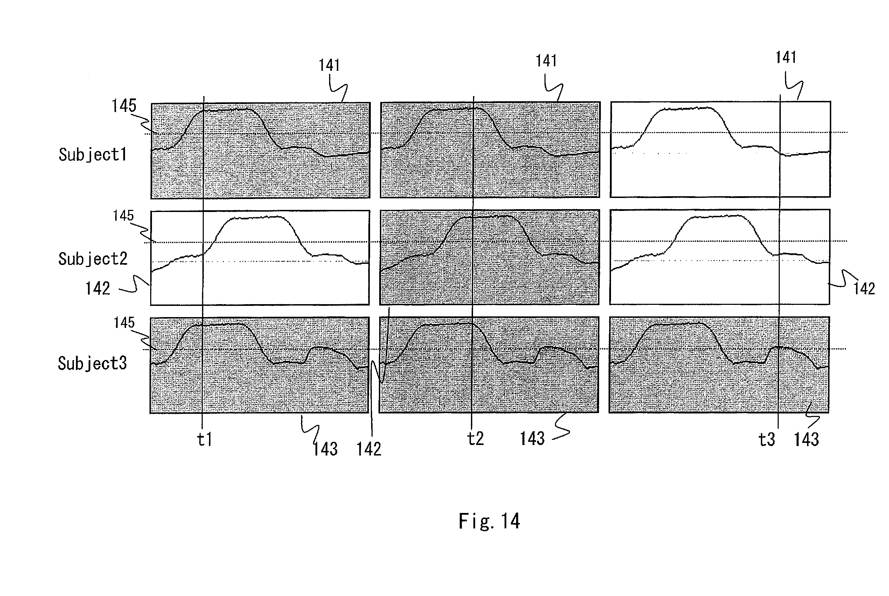

The second embodiment is an embodiment in which topography image or waveform graphs of noted channels for biophotonic measurement data of a plurality of subjects are juxtapositionally displayed with emphasis. The second embodiment will be explained with reference to Fig. 14. Fig. 14 is an explanatory drawing showing an example of distinguishable display of waveform graphs of a plurality of subjects in rows. More precisely, waveform graphs 141, 142, and 143 for one typical channel based on biophotonic measurement data obtained by imposing the same task on three subjects "Subject 1", "Subject 2", and "Subject 3" and stored in the measurement result storage part 81 are juxtapositionally displayed. In Fig. 14, three of the waveform graphs 141 of "Subject 1" are transversely displayed in the top row. Three of the waveform graphs 141 are all the same. Similarly, three of the waveform graphs 142 of "Subject 2" are transversely displayed in the middle row, and three of the waveform graphs 143 of "Subject 3" are transversely displayed in the bottom row.

At three time points of t1, t2, and t3 in the waveform graphs 141, 142, and 143, the extraction processing part 84 judges that there is a reaction when the hemoglobin amount exceeds a threshold value 145 set by the activation condition setting part 71, or judges that there is no reaction when the hemoglobin amount is not higher than the threshold value, and the distinguishable display processing part 85 realizes distinguishable display with a relatively darker background color when there is a reaction, and with a relatively lighter background color when there is no reaction. An example of interpretation using waveform graphs distinguishably displayed as described above is mentioned below to demonstrate the effectiveness of this function.

In "Subject 1", there is a reaction at the time point of t1, and at the time point t2, the reaction is maintained. When the time advances to the time point t3, which is a noted time point, there is no longer a reaction. This state is assumed to be a typical reaction transition.

On the other hand, "Subject 2" does not satisfy the condition at the time point t1 (no reaction). When the time advances to t2, there is a reaction, and when the time advances to t3, there is no longer a reaction. On the basis of these results, it can be seen that "Subject 2" slowly shows the reaction to the task. Definitely judging whether there is a reaction or not at the time point t1 and further displaying the result in an easily understandable manner make the judgment easier.

Furthermore, "Subject 3" shows the same tendency as that of "Subject 1" at the time points t1 and t2. However, a unique reaction is observed at t3. It is considered that, if the conventional result display scheme is used, the reaction at the time point t3 is highly possibly overlooked among the results of many channels by a measurement operator, because the Subject 3 showed the typical tendency for the reaction before the time point t3. According to this embodiment, there are enabled mechanical extraction of channels satisfying the condition and display thereof without depending on visual inspection or subjectivity of a measurement operator, and therefore such an oversight as mentioned above can be prevented.

### <Third embodiment>

The third embodiment is an embodiment in which the reaction state is recorded. The "Save" button 76 is provided in the condition setting screen of Fig. 8. If this button is operated by a measurement operator, the result data storage part 89 records the result data (waveform or topography image) at the time of operation of the button.

Furthermore, the result data storage part 89 displays the times at which the result data are recorded in ascending order in the recorded time display part 761 of the condition setting screen 70. An arbitrary time displayed in the recorded time display part 761 is selected, a play back part 810 reads out the result data recorded at that time and display them. Three types of examples concerning use of the functions of the data storage part 89 and the play back part 810 are shown below.

The first example is a method of performing the recording according to the state of one or more representative channels. To record the result data at a plurality of time points at which characteristic change of data is observed for the representative channels, and successively display them is effective for seeing the results. For example, results at the time point at which a reaction is observed for a noted channel set with the "Selection button" 74 is first recorded, then the time point at which a steady state is attained via a transition state is recorded, the time point at which the reaction begins to decrease is further recorded, and the time point at which the waveform is stabilized to be the same as that of resting state is finally recorded. By confirming these time points and further successively confirming the whole waveforms at the time points afterward, what kinds of tendencies is shown by the channels other than the noted channels at the noted time points for the noted channels can be easily confirmed, and thus it is made possible to generally see the brain activities. In such confirmation, new characteristics not expected by the measurement operator may be found for the other channels.

Alternatively, there is also contemplated a way of use in which whole waveforms are confirmed with successively changing the time by operating the time operating bars 724, and time points at which certain characteristic results are observed during the operation and waveforms at the time points are marked.

The second example is a method of setting several kinds of the extraction conditions for a certain time point, and performing recording for each condition. For example, when an activation condition based on ratio at the lapsed time 722 of 10 [s]is used, the ratio of the activation condition to be set with the activation condition setting part 71 is changed to be 60 [%], 70 [%], and 80 [%], and the result data obtained for these ratios are compared. This method can also be used together with the method of the first example. First, the ratio as the activation condition is set to be to 60 [%] with the activation condition setting part 71, time is changed by operating the time operating bars 724, and time point at which a plurality of channels for which reaction is observed appear is recorded. Then, at that time point, the ratio as the activation condition is elevated to be 70 [%] and 80 [%], and recording is performed for each condition. In such a manner, a reactive site is more precisely specified.

The third example is a method of using a plurality of kinds of tasks, and comparing reactions observed for the tasks. For example, when a finger tapping task is alternately imposed two times each for left and right, the time during the first left finger task at which the reaction state is best represented is recorded, and then similar recording is also performed for the reaction state during the first right finger task. By comparing two kinds of times and two kinds of reaction states, characteristics of the reaction to the tapping task observed for right and left fingers can be easily understood. Alternatively, for only the left finger (right finger) task, the reaction states observed during the first repetition and second repetition may be compared.

According to the aforementioned methods, activities of a measurement part (mainly brain) can be "generally seen in time series" by observing "time until data start to change" and "data change" concerning change of hemoglobin concentration obtained with as many as several tens of channels of the biophotonic measurement device.

### <Fourth embodiment>

The fourth embodiment is an embodiment in which, when the extraction processing part 84 judged that "there is a reaction", the distinguishable display processing part 85 displays measurement channels for which the reaction is observed by using color shade corresponding to the hemoglobin amount for the display color.

In the first, second, and third embodiments, the state of "there is a reaction" is displayed with three kinds of states, i.e., (1) significant increase compared with the previously measured value, (2) maintenance of the previously measured value, and (3) significant decrease compared with the previously measured value. However, states after the condition is satisfied may be displayed in detail similarly to the conventional topography display. This is realized by setting the time frame to be "0 (zero)" with the time frame for change calculation setting part 73. If "0 (zero)" is set with the time frame for change calculation setting part 73, the channels that satisfy the extraction condition in the enlarged topography image of Fig. 8 are thereafter displayed with precise shade of color corresponding to the value at that time point as in Fig. 4, i.e., colors corresponding to biological metabolite concentrations. Also in such a waveform map as mentioned in Fig. 6, for the channels that satisfy the extraction condition, the whole background of the graph is thereafter depicted with precise shade of color similar to that of Fig. 4.

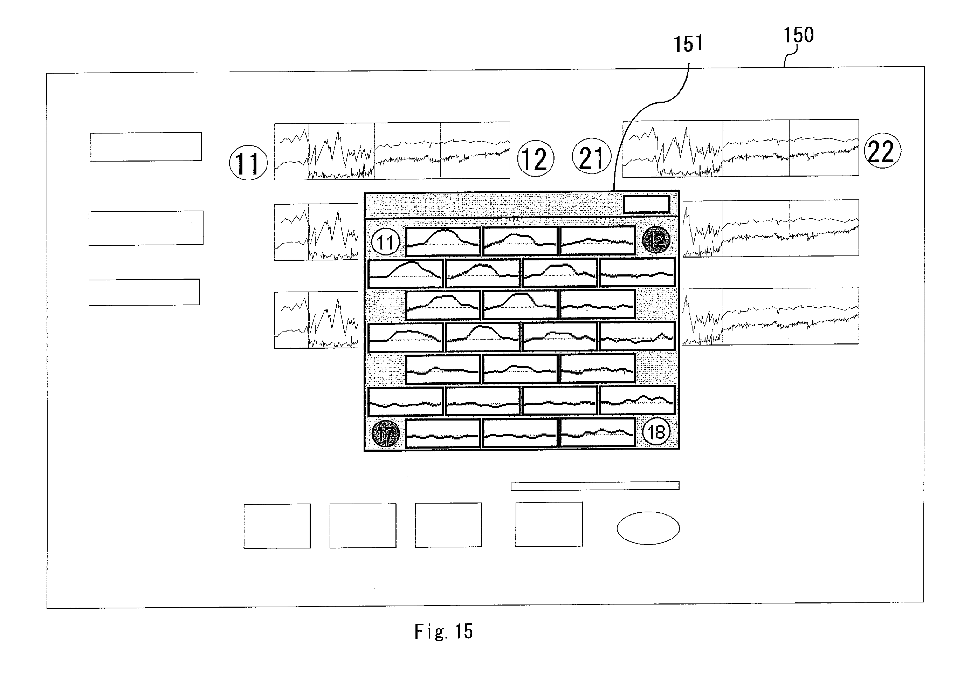

Fig. 15 is a schematic drawing showing an example of an actual display screen. The screen explained for the additional function is superimposingly displayed on a conventional waveform map. In the example of Fig. 15, a waveform map 151 utilizing the distinguishable display of Fig. 10 as the screen explained for the additional function is superimposed on a conventional screen 150 in which various kinds of buttons and graphs indicating time series change of biological metabolite concentration for every channel are displayed. By superimposingly displaying the conventional screen 150 and the screen 151 explained for the additional function as described above, channels satisfying the extraction condition and states thereof can be confirmed with confirming the familiar conventional screen 150. The example of the display screen shown in Fig. 15 is a mere example, and the combination of the conventional screen 150 and the screen 151 explained for the additional function may be arbitrarily employed.

According to this embodiment, measurement channels for which reaction is observed at a certain time can be more easily distinguished compared with the conventional method.

### Denotation of Reference Numerals

1: Wearing part, 2: light irradiation probe group, 3: light detection probe group, 4: optical fiber group for light irradiation, 5: optical fiber group for detection, 6: main part of measurement device, 7: irradiation and measurement part, 8: display control part, 9: monitor, 10: biophotonic measurement device, 11: holder

## Claims

1. A biophotonic measurement device comprising an irradiation and measurement part for irradiating near-infrared light on a measurement site of a subject, measuring transmitted light intensities of the near-infrared light at a plurality of measurement points of the subject, and outputting signals corresponding to the transmitted light intensities at the measurement points as biophotonic measurement data of the measurement points, and
a display controller for displaying result data indicating distribution of a biological metabolite concentration of the subject based on the biophotonic measurement data, wherein:
the display controller comprises a result data generation part for generating the result data by using the biophotonic measurement data,
an extraction condition setting part which sets an arbitrary extraction condition for the biophotonic measurement data,
an extraction processing part which judges whether the biophotonic measurement data satisfy the extraction condition or not and performing extraction of the biophotonic measurement data on the basis of results of the judgment, and
a distinguishable display processing part which distinguishably displays the result data on the basis of whether the biophotonic measurement data satisfy the extraction condition or not.

2. The biophotonic measurement device according to claim 1, wherein:
the extraction condition setting part sets at least one of a ratio relative to a standard value of the biological metabolite concentration or value of the biological metabolite concentration, time passed from a standard time corresponding to a time point at which the subject starts a task, and a time frame used for calculation of change of the biological metabolite concentration, as the extraction condition.

3. The biophotonic measurement device according to claim 2, wherein:
the extraction condition setting part sets a ratio relative to a standard value of the biological metabolite concentration or value of the biological metabolite concentration, and a time frame used for calculation of change of the biological metabolite concentration as the extraction condition,
the extraction processing part judges in which state the subject is among states of:
a maintenance reaction where, during the time frame, ratio relative to the standard value or value of the biological metabolite concentration of the subject is not lower than the ratio relative to the standard value or value of biological metabolite concentration defined as the extraction condition, and the ratio or value continues,
an increasing reaction where, during the time frame, ratio relative to the standard value or value of the biological metabolite concentration of the subject is not lower than the ratio relative to the standard value or value of biological metabolite concentration defined as the extraction condition, and the biological metabolite concentration increases,
a decreasing reaction where, during the time frame, ratio relative to a standard value or value of the biological metabolite concentration of the subject is not lower than the ratio relative to the standard value or value of the biological metabolite concentration defined as the extraction condition, and the biological metabolite concentration decreases, and
no reaction where, during the time frame, a state that ratio relative to the standard value or value of the biological metabolite concentration of the subject is lower than the ratio relative to the standard value or value of the biological metabolite concentration defined as the extraction condition continues, and
the distinguishable display processing part distinguishably displays the result data by using display modes corresponding to the aforementioned states.

4. The biophotonic measurement device according to claim 1, wherein:
the result data generation part generates an enlarged topography image in which geometric figures surrounding the measurement points of the biological metabolite concentration of the subject are displayed by using one display mode for displaying whether the extraction condition is satisfied or not.

5. The biophotonic measurement device according to claim 1, wherein:
the result data generation part generates waveform graphs indicating temporal change of the biological metabolite concentration for the measurement points, and
the distinguishable display processing part distinguishably displays waveform graphs satisfying the extraction condition and waveform graphs not satisfying the extraction condition.

6. The biophotonic measurement device according to claim 5, wherein:
the result data generation part generates a waveform map in which waveform graphs corresponding to the measurement points are arranged in accordance with arrangement of the measurement points,
the extraction processing part judges whether the extraction condition is satisfied or not for the waveform graphs, and
the distinguishable display processing part distinguishably displays the waveform graphs in the waveform map on the basis of whether the extraction condition is satisfied or not.

7. The biophotonic measurement device according to claim 5, wherein:
a time specification part for specifying an arbitrary time on the waveform graphs is further provided,
the extraction processing part judges whether the extraction condition is satisfied or not at the specified time, and
the distinguishable display processing part distinguishably displays the whole of the waveform graph.

8. The biophotonic measurement device according to claim 5, wherein:
the extraction processing part divides one of the waveform graphs into a plurality of regions, and judges whether the extraction condition is satisfied or not for each region, and
the distinguishable display processing part distinguishably displays the regions of the one waveform graph on the basis of whether the extraction condition is satisfied or not.

9. The biophotonic measurement device according to claim 1, wherein:
when the extraction processing part judges that the extraction condition is not satisfied, the distinguishable display processing part displays the result data by using a display mode corresponding to no satisfaction, and when the extraction processing part judges that the extraction condition is satisfied, the distinguishable display processing part displays the result data by using a display mode corresponding to the biological metabolite concentration.

10. The biophotonic measurement device according to claim 1, wherein:
a measurement result storage which stores biophotonic measurement data obtained by making different subjects perform the same task is further provided,
the result data generation part generates the result data for the same time based on the time when the same task is started for each subject as the standard,
the extraction processing part judges whether the extraction condition is satisfied or not at that same time, and
the distinguishable display processing part displays juxtapositionally the result data generated for the different subjects, and distinguishably displays the result data on the basis of whether the extraction condition is satisfied or not.

11. The biophotonic measurement device according to claim 1, which further comprises:
a first selection part for calculating integral value of the biological metabolite concentration of the subject during execution of the task, and selecting biophotonic measurement data giving such an integral value not lower than a predetermined value, and
an emphasized display processing part for displaying result data based on the selected biophotonic measurement data with emphasis.

12. The biophotonic measurement device according to claim 1, which further comprises:
a second selection part for selecting an arbitrary measurement point and selecting biophotonic measurement data of the measurement point, and
an emphasized display processing part for displaying result data based on the selected biophotonic measurement data with emphasis.

13. The biophotonic measurement device according to claim 1, which further comprises:
a result data storage which stores at least one or more of display screens displaying the result data at an arbitrary time point, and
a play back part which plays back a display screen stored in the result data storage part.

14. A method for operating a biophotonic measurement device comprising the step of generating result data indicating distribution of biological metabolite concentration in a subject by using biophotonic measurement data obtained with a biophotonic measurement device comprising an irradiation and measurement part for irradiating near-infrared light on a measurement site of a subject, measuring transmitted light intensities of the near-infrared light at a plurality of measurement points of the subject, and outputting signals corresponding to the transmitted light intensities at the measurement points as the biophotonic measurement data of the measurement points, which comprises:
the step of setting an arbitrary extraction condition for the biophotonic measurement data,
the step of judging whether the set extraction condition is satisfied or not, and performing extraction of the biophotonic measurement data on the basis of results of the judgment, and
the step of distinguishably displaying the result data on the basis of whether the extraction condition is satisfied or not.

15. A method for analyzing and displaying biophotonic measurement data, which comprises:
the step of reading biophotonic measurement data of a subject,
the step of generating result data indicating distribution of biological metabolite concentration in the subject by using the biophotonic measurement data,
the step of setting an arbitrary extraction condition for the biophotonic measurement data,
the step of judging whether the set extraction condition is satisfied or not, and performing extraction of the biophotonic measurement data on the basis of result of the judgment, and
the step of distinguishably displaying the result data on the basis of whether the extraction condition is satisfied or not.
